(19) Europäisches Patentamt
European Patent Office
Office européen des brevets

(11) Numéro de publication : **0 394 078 B1**

(12) # FASCICULE DE BREVET EUROPEEN

(45) Date de publication du fascicule du brevet : **16.06.93 Bulletin 93/24**

(51) Int. Cl.[5] : **A61K 7/00,** A61K 7/06

(21) Numéro de dépôt : **90400515.4**

(22) Date de dépôt : **23.02.90**

(54) **Composition cosmétique de traitement des cheveux contenant une microdispersion de cire.**

(30) Priorité : **24.02.89 LU 87457**

(43) Date de publication de la demande : **24.10.90 Bulletin 90/43**

(45) Mention de la délivrance du brevet : **16.06.93 Bulletin 93/24**

(84) Etats contractants désignés : **AT BE CH DE DK ES FR GB GR IT LI NL SE**

(56) Documents cités :
**EP-A- 0 045 008**
**EP-A- 0 101 007**
**EP-A- 0 167 825**
**EP-A- 0 283 247**
**DE-A- 1 794 088**
**FR-A- 2 222 998**
**FR-A- 2 542 008**

(73) Titulaire : **L'OREAL**
**14, Rue Royale**
**F-75008 Paris (FR)**

(72) Inventeur : **Vanlerberghe, Guy**
**40, rue du Général de Gaulle**
**F-77410 Montjay-La-Tour (FR)**
Inventeur : **Nicolas-Morgantini, Luc**
**5, rue du Vignet**
**F-60810 Rully (FR)**
Inventeur : **Lety, Alain**
**9, rue de Metz**
**F-7400 Lagny-Sur-Marne (FR)**

(74) Mandataire : **Tonnellier, Jean-Claude et al**
**Cabinet Nony & Cie. 29, rue Cambacérès**
**F-75008 Paris (FR)**

EP 0 394 078 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet l'utilisation, comme compositions cosmétiques ou supports de compositions cosmétiques pour cheveux, de microdispersions de cire, ainsi qu'un procédé de traitement cosmétique pour cheveux à l'aide de ces compositions.

On sait que les cires, dont l'utilisation en cosmétologie est très ancienne, sont des substances naturelles (animales ou végétales) ou synthétiques, solides à la température ordinaire (21°C), ayant généralement une certaine plasticité, qui sont insolubles dans l'eau, solubles dans les huiles, et qui sont capables de former des films hydrofuges. Sur la définition des cires et leurs utilisations en cosmétologie, on peut citer par exemple P.D. Dorgan, Drug and Cosmetic Industry, Décembre 1983, pages 30-33, et Handbook of Cosmetic Science, H.W. Hibbot ed., Pergamon Press, Oxford (1963) page 60. Dans les préparations capillaires, l'utilisation la plus classique est celle de préparations semi-solides appelées pommades pour cheveux ou brillantines solides. Dans de telles compositions, les cires sont utilisées en mélange notamment avec des proportions importantes d'huiles diverses ; voir par exemple E.W. Flick, "Cosmetic and Toiletry Formulations" Ed. Noyes Publications-New Jersey-USA (1984) pages 271-288.

Dans la demande de brevet allemand 3.534.733, on a décrit des compositions cosmétiques moussantes et limpides pouvant contenir une huile et éventuellement de 0,05 à 1% de cire. Dans ces compositions, l'huile, et la cire éventuellement présente, se trouvent solubilisées. La présence d'huile et/ou de cire dans ces compositions a pour but de regraisser la peau et d'éviter ainsi le dégraissage trop important de la peau entraîné par l'emploi des tensio-actifs anioniques.

Le document DE-A-1794088 décrit des compositions cosmétiques sous forme d'émulsions eau-dans-l'huile ou huile-dans-l'eau, pouvant contenir une cire microcristalline en solution dans la phase huileuse.

On sait par ailleurs qu'il est possible d'obtenir avec certaines huiles des microémulsions et avec certaines cires des microdispersions stables et diluables à l'eau indéfiniment, sans agrégation ni sédimentation des particules en suspension. Les microdispersions de cire sont obtenues par fusion de la cire en présence d'un tensioactif anionique ou non-ionique, et éventuellement d'une partie de l'eau, puis addition progressive d'eau chaude avec agitation. On observe la formation intermédiaire d'une émulsion du type eau-dans-l'huile, suivie d'une inversion de phase avec obtention finale d'une émulsion du type huile-dans-l'eau. Au refroidissement, on obtient une microdispersion stable de particules colloïdales solides de cire ; voir par exemple "Microemulsions Theory and Practice", L.M. Prince Ed., Academic Press (1977) pages 21-32.

Ces microdispersions de cire sont utilisées notamment pour faire briller les articles en cuir et les revêtements de sol en matière plastique.

On a maintenant découvert que de telles microdispersions de cire peuvent être utilisées notamment comme lotions coiffantes conférant du volume à la coiffure, plus d'épaisseur aux cheveux et notamment aux cheveux fins et mous; ces lotions permettant d'obtenir des cheveux disciplinés et gainés jusqu'à leurs pointes qu'elles rendent lisses. Elles présentent également la propriété surprenante de ne pas conférer aux cheveux un aspect gras mais, au contraire, d'en retarder l'apparition, bien qu'elles contiennent comme ingrédient actif principal une cire, c'est-à-dire une substance classée parmi les matières grasses.

La présente invention a donc pour objet l'utilisation, comme composition cosmétique ou support de composition cosmétique pour cheveux, d'une composition fluide non moussante contenant une dispersion de cire dans un véhicule liquide, dont la phase dispersée est une microdispersion stable de particules solides de dimensions inférieures à 500nm, lesdites particules contenant une cire ou un mélange de cires, et éventuellement une huile à une concentration pouvant aller jusqu'à -30% en poids, par rapport à la cire, ladite cire ou ledit mélange ayant un point de fusion finissante supérieur à 60°C et inférieur à 100°C et étant capable de former une microdispersion telle que définie ci-dessus, ladite composition contenant, en poids, de 0,1 à 40% de cire, de 0,01 à 25% d'au moins un agent émulsionnant non ionique ou anionique et au moins 35% d'eau, le rapport pondéral cire/émulsionnant pouvant varier de 1 à 30.

La cire ou le mélange de cires utilisé selon l'invention doit donc être capable de donner, en association avec des agents émulsionnants non ioniques et/ou anioniques, selon le procédé décrit ci-dessus, des microdispersions stables ayant des dimensions de particules inférieures à 500nm. Les cires ou mélanges de cires utilisables peuvent être choisis par de simples expériences de routine. Bien entendu, la microdispersion de cire utilisée selon l'invention ne contient pas d'agent tensioactif cationique.

Dans des modes de réalisation particuliers, les compositions utilisées selon l'invention peuvent encore présenter les caractéristiques suivantes, prises isolément ou en combinaison:

- la cire est une cire choisie parmi la cire de Carnauba, la cire de Candelilla, la cire d'Alfa et leurs mélanges ;
- la cire contient, outre de la cire de Carnauba ou de la cire de Candelilla ou leurs mélanges, une autre cire ou un mélange d'autres cires, par exemple une cire de paraffine, l'ozokérite, la cire de jojoba hydrogénée, la cire d'abeille éventuellement estérifiée, la cire de riz, ou un céramide ; la proportion pondérale de cire de Carnauba et/ou de Candelilla, dans de tels mélanges, est de préférence supérieure ou égale à 50% ;

- dans les compositions cosmétiques, la proportion de cires est par exemple de 0,1 à 20%, notamment de 1 à 20%, et en particulier de 1 à 10%;
- l'agent émulsionnant a une concentration de 0,1 à 10% ;
- ledit agent émulsionnant non ionique est un tensio-actif polyalcoxylé et/ou polyglycérolé ;
- ledit agent émulsionnant est un tensio-actif anionique ;
- le véhicule liquide contient de 80 à 100% d'eau, par rapport au poids de la phase liquide;
- le véhicule liquide est constitué par de l'eau ;
- au moins un composé amphiphile (non émulsionnant des cires) tel que le cholestérol, les alcools gras contenant au moins 12 atomes de carbone, etc ..., peut être associé à la cire ; la concentration en composés amphiphiles peut aller jusqu'à 30 % (notamment jusqu'à 10 %) en poids par rapport à la cire ou au mélange de cires ;
- Une huile ou un mélange d'huiles (notamment celles qui sont mentionnées dans la partie expérimentale ci-après), peut être associé à la cire ; la concentration de l'huile peut aller jusqu'à 30 % (notamment jusqu'à 10 %) en poids par rapport à la cire ou au mélange de cires ; parmi les compositions selon l'invention, on citera notamment celles qui ne contiennent pas d'huile ;
- la composition peut en outre contenir, le cas échéant, au moins un ingrédient actif liposoluble ; parmi les ingrédients actifs liposolubles, on peut citer notamment des colorants liposolubles ou des filtres solaires (substances capables de protéger la peau et/ou les cheveux contre les effets nocifs du rayonnement ultraviolet) solubles ;
- la concentration du ou des ingrédients actifs liposolubles, lorsqu'ils sont présents, peut aller jusqu'à 30 % (généralement jusqu'à 10 %) en poids par rapport au poids de cire ou de mélange de cires ;
- la proportion pondérale de cire, et de composés amphiphiles non émulsionnants éventuellement présents, dans les particules est généralement supérieure à 90%, par rapport au poids des particules, et est le plus souvent supérieure à 95%, le reste étant constitué par les huiles et/ou les ingrédients liposolubles éventuellement présents (non compris les agents émulsionnants) ;
- le rapport pondéral cire/émulsionnant peut varier dans la gamme de 1 à 20, notamment de 2 à 10.

Les cires végétales de Carnauba (extraite de Copernicia Cerifera), de Candelilla (extraite de Euphorbies Cerifera et de Pedilantus Pavonis), et d'Alfa (extraite de Stipa Tenacissima) sont des produits commerciaux.

Il est également possible de préparer des compositions cosmétiques ou supports de compositions cosmétiques en utilisant des mélanges commerciaux de cires auto-émulsionnables contenant la cire et les émulsionnants. On peut utiliser par exemple la cire commercialisée sous la dénomination CIRE AUTO LUSTRANTE OFR par TISCCO, Bobigny (France), qui contient des cires de Carnauba et de paraffine, en association avec des agents émulsionnants non ioniques, ou la cire auto-émulsionnable commercialisée sous la dénomination CERAX A.O. 28/B par LA CERESINE, Marseille (France), qui contient de la cire d'Alfa en association avec un émulsionnant non ionique. Ces mélanges commerciaux permettent de préparer des microdispersions de cires par addition d'eau selon le procédé décrit ci-dessus.

Les céramides sont les principaux lipides constitutifs des espaces intercornéocytaires du stratum corneum. Ils ont été décrits, en particulier par Downing dans Science 1982 p 1261-2 vol.18. Les céramides sont utilisés notamment dans les compositions cosmétiques comme agents anti-vieillissement et comme agents hydratants ; voir par exemple la demande de brevet japonais 87.176907. Dans les compositions capillaires, ils agissent comme agents de protection du cheveu ; voir par exemple la demande de brevet européen 0278505.

Ces céramides sont difficiles à disperser dans les compositions cosmétiques. Grâce à la présente invention, il est possible de les disperser à des concentrations élevées.

Les colorants liposolubles éventuellement présents dans la composition sont par exemple :

- Nitro 1-amino-3 isopropyl 4 aniline,
- Nitro 1-méthyl-2 méthylamino 3 méthyl 4 aniline,
- Nitro 3 butylamino 4 phénol,
- Hydroxy-4 méthyl-3 phénylazo benzène,
- Le produit de formule :

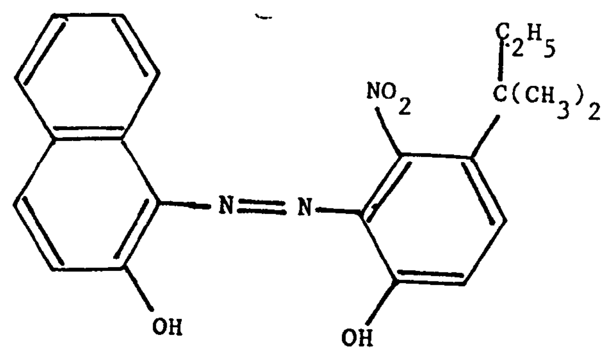

Parmi les filtres solaires liposolubles éventuellement présents dans la composition de l'invention, on peut citer notamment les suivants, qui sont disponibles dans le commerce :

- le 3-benzylidène-d,1-camphre,
- le 3-(4'-méthylbenzylidène)-d 1-camphre,
- le 4-(diméthylamino)-benzoate d'amyle,
- le p-méthoxycinnamate d'amyle et d'isoamyle,
- le salicylate de méthyle,

L'un des avantages de la composition de l'invention est de permettre l'utilisation de ces ingrédients liposolubles en milieu aqueux.

Les tensio-actifs anioniques utilisés ont de préférence une balance lipophile-hydrophile (HLB) pouvant aller de 10 à 40. Ce sont notamment les sels d'acides gras (par exemple sels alcalins ou sels organiques tels que les sels d'amines), lesdits acides gras ayant par exemple de 12 à 18 atomes de carbone et pouvant comporter une double liaison comme dans le cas de l'acide oléique; les sels alcalins ou sels de bases organiques des acides alkyl-sulfuriques et alkyl-sulfoniques ayant 12 à 18 atomes de carbone, des acides alkyl-arylsulfoniques dont la chaîne alkyle contient de 6 à 16 atomes de carbone, le groupement aryle étant par exemple un groupement phényle. Ce sont également les éthers-sulfates, en particulier les produits de sulfatation des alcools gras et alkylphénols polyalcoxylés, dans lesquels la chaîne aliphatique comporte de 6 à 20 atomes de carbone et la chaîne polyalcoxylée de 1 à 30 motifs oxyalkylène, en particulier oxyéthylène, oxypropylène ou oxybutylène.

Tous ces tensio-actifs anioniques sont bien connus et beaucoup d'entre eux sont des produits commerciaux.

Les tensio-actifs non ioniques sont notamment les acides gras ou les amides d'acides gras polyalcoxylés et/ou polyglycérolés ; les esters d'acides gras et de polyols polyalcoxylés et/ou polyglycérolés ; les alcools gras ou les alkylphénols polyalcoxylés et/ou polyglycérolés; les alcanediols ou alcènediols-1,2 ou -1,3 polyalcoxylés et/ou polyglycérolés ; et les alkyléthers d'alcanediols ou alcènediols -1,2 ou -1,3 polyalcoxylés et/ou polyglycérolés. Les acides ou alcools gras, éventuellement insaturés, ont par exemple 12 à 24 atomes de carbone, la chaîne alkyle des alkylphénols a par exemple 6 à 16 atomes de carbone, les alcanediols ou alcènediols ont de 9 à 24 atomes de carbone, l'alkyle des alkyléthers a de 4 à 20 atomes de carbone, et le nombre de motifs oxyalkylène ou de motifs ($CH_2CHOH$ $CH_2O$) peut aller de 2 à 40.

Les dérivés non ioniques polyalcoxylés sont notamment des dérivés polyoxyéthylénés, éventuellement polyoxypropylénés.

Les acides gras polyalcoxylés sont des produits commerciaux, notamment les produits vendus sous la marque Myrj.

Les esters d'acides gras et de polyols polyoxyéthylénés pour lesquels le polyol est le sorbitol sont des produits connus (Polysorbate et produits commercialisés sous la marque Tween).

Les alcools gras polyoxyéthylénés sont des produits commerciaux, notamment ceux vendus sous la marque Brij.

Les alcools gras polyglycérolés, les alcanediols ou alcènediols polyglycérolés, ou les alkyléthers d'alcanediols ou d'alcènediols polyglycérolés peuvent être préparés par exemple selon les procédés décrits dans les brevets français 1.477.048, 2.025.681, 2.091.516 et 2.465.780, ou selon des procédés analogues.

Les acides gras ou amides d'acides gras polyglycérolés sont notamment décrits dans le brevet français 1.484.723 ou sont encore des produits commerciaux tels que ceux vendus sous la marque PLUROL (Gattefossé) ou DREWPOL (Stefan Company).

Les compositions cosmétiques obtenues selon l'invention peuvent aussi contenir un ou plusieurs ingrédients secondaires usuels tels que des agents épaississants, des agents stabilisants, des parfums ou des

agents conservateurs.

Les compositions sans agent épaississant sont des lotions fluides. Les compositions avec agent épaississant sont des lotions ou des gels fluides.

Les agents épaississants sont plus particulièrement choisis parmi les acides polyacryliques réticulés par un agent polyfonctionnel tels que les produits vendus sous la dénomination CARBOPOL par la Société Goodrich, comme les Carbopols 910, 934, 934P, 940, 941, 1342, ou des dérivés cellulosiques comme l'hydroxyméthylcellulose, la carboxyméthylcellulose, l'hydroxybutylcellulose, l'hydroxypropylcellulose) et plus particulièrement l'hydroxyéthylcellulose, tels que les produits vendus sur la dénomination "NATROSOL" (150, 250) par la société HERCULES ou "CELLOSIZE" (QP et WP) par la société UNION CARBIDE, la méthylhydroxypropyl cellulose, en particulier les produits vendus sous la dénomination "METHOCEL" (E, F, J, K) par la société DOW CHEMICAL ou des hétérobio-polysaccharides tels que par exemple les gommes de xanthane commercialisées sous les marques "KELTROL" et "KELZAN" par la société KELCO, "RHODOPOL" et "RHODIGEL" par la société RHONE POULENC, ou "ACTIGUM" par la société CECA/SATIA.

Lorsqu'on utilise des épaississants, ceux-ci sont de préférence choisis parmi les Carbopols et utilisés de préférence à une concentration telle que la viscosité de la composition soit au plus égale à 25 poises (soit 2,5 Pa.s) environ à 25°C (viscosimètre Contraves; corps de mesure n°3; temps de rotation 10 minutes, à 200 tours/min.).

Les parfums utilisables sont des parfums usuels, solubles dans la cire ou dispersibles ou solubles dans l'eau, en particulier ceux qui sont dispersibles ou solubles dans l'eau. Ils sont généralement utilisés à une concentration ne dépassant pas 5%.

Comme agents stabilisants, on peut citer les esters phosphoriques d'alcools gras. Ils sont utilisés généralement à une concentration inférieure à 1%.

Les agents conservateurs sont par exemple l'acide parahydroxybenzoïque, ses sels et esters, l'acide sorbique et ses sels, la diméthyloldiméthylhydantoïne et les dérivés d'imidazolidinyl urée. Ils sont utilisés aux concentrations efficaces usuelles.

Le pH des compositions obtenues selon l'invention peut varier de 3 à 10. Le pH peut éventuellement être ajusté à l'aide d'un agent modificateur de pH usuel.

L'invention a également pour objet l'utilisation de microdispersions de cires, telles que définies précédemment, dans la préparation d'une composition cosmétique fluide non moussante telle que décrite ci-dessus.

Les compositions selon l'invention sont obtenues par formation à chaud d'une microémulsion. Plus précisément, ces compositions sont obtenues par un procédé principalement caractérisé par le fait que l'on chauffe la cire et l'émulsionnant à une température supérieure à la température de fusion de la cire et non supérieure à 100°C, éventuellement en présence d'une partie de l'eau, jusqu'à fusion complète de la cire, que l'on ajoute progressivement l'eau, ou le restant de l'eau, portée à une température au moins égale à ladite température, en agitant, jusqu'à formation d'une microémulsion de cire dans une phase continue aqueuse, qui par refroidissement jusqu'à la température ambiante conduit à une microdispersion de cire.

Les ingrédients secondaires éventuellement présents dans la composition sont ajoutés selon les cas soit dans les produits de départ, soit dans la composition terminée.

Les ingrédients hydrosolubles non volatils peuvent être ajoutés éventuellement dans l'eau utilisée pour réaliser la microdispersion.

Les ingrédients liposolubles sont généralement ajoutés à la cire avant la réalisation de la microdispersion.

Les compositions obtenues selon l'invention peuvent notamment être utilisées comme lotions coiffantes et aussi comme lotions destinées à améliorer l'aspect des cheveux chez les sujets ayant les cheveux gras.

Elles peuvent être appliquées sur cheveux secs ou mouillés et propres ou sales, et aussi avant ou après un shampooing. Elles peuvent être rincées ou non rincées et être appliquées quotidiennement.

Lorsqu'elles sont appliquées avant ou après un shampooing, l'application étant suivie ou non d'un rinçage à l'eau, elles disciplinent les cheveux et communiquent à la coiffure de la tenue et du volume. En outre, elles retardent le phénomène de regraissage des cheveux observé chez les sujets ayant les cheveux gras.

Pour éviter ce phénomène de regraissage, on peut appliquer la composition de l'invention sur les cheveux séchés après lavage, et en particulier sur la partie des cheveux voisine de la racine. Dans ce cas, la composition n'est pas rincée. On observe que, malgré l'absence de rinçage, la composition ne communique pas aux cheveux un toucher poisseux, et ne provoque pas de phénomène de collage des cheveux.

Malgré la présence d'une cire dans la composition, aucun aspect gras n'est communiqué aux cheveux, même en l'absence de rinçage. En outre, malgré la présence d'une forte proportion d'eau dans la composition, le séchage ne pose pas de problèmes et s'effectue rapidement.

Quand la composition contient un agent colorant liposoluble, elle peut être utilisée comme composition de teinture pour cheveux.

Les compositions sous forme de gels sont utilisées comme gels de coiffage.

Il convient encore de noter que les supports de compositions selon l'invention, qui sont diluables en toutes proportions avec de l'eau, peuvent être réalisés sous forme de supports de compositions, éventuellement concentrés contenant par exemple de 1 à 40% en poids de cire. Ils se présentent sous la forme de compositions fluides.

Les supports de compositions concentrés peuvent être dilués au moment de l'emploi, pour obtenir une concentration en cire pouvant aller par exemple de 0,1 à 10% en poids.

On peut également ajouter (après dilution, dans le cas des compositions concentrées) des ingrédients secondaires.

Les supports de composition cosmétique obtenus selon l'invention sont donc d'une part les microdispersions concentrées mentionnées ci-dessus, à diluer au moment de l'emploi, et d'autre part les microdispersions non concentrées (contenant au moins l'eau, la cire et le tensio-actif) auxquelles on peut ajouter, au moment de l'emploi, les ingrédients secondaires définis ci-dessus.

L'invention a également pour objet un procédé de traitement cosmétique des cheveux destiné à améliorer la tenue et le volume de la coiffure et/ou à supprimer ou à retarder l'apparition de l'aspect gras des cheveux, caractérisé par le fait que l'on applique, au moins sur la partie des cheveux voisine de la racine, en quantité suffisante pour imprégner les cheveux ou les parties de cheveux à traiter, une composition telle que définie précédemment, après dilution éventuelle pour que la proportion pondérale de cire dans la composition soit de 0,1 à 20%. La dilution éventuelle est bien entendu effectuée avec de l'eau qui peut contenir en outre des ingrédients hydrosolubles de la composition désirée.

Ce procédé de traitement cosmétique des cheveux est mis en oeuvre comme cela a été indiqué plus haut.

Les exemples suivants illustrent l'invention sans toutefois la limiter.

Les compositions décrites dans les exemples de préparation A à L peuvent être utilisées comme supports de composition cosmétique, ou comme lotion pour cheveux, après dilution, si nécessaire, et après éventuel ajustement du pH.

## EXEMPLES DE PREPARATION DE MICRODISPERSIONS DE CIRE

### EXEMPLE A

On porte à 90°C un mélange de 10g de cire de Carnauba et de 1,84g d'un tensio-actif non ionique résultant de la polyaddition de 3 moles de glycidol sur 1 mole de nonylphénol, mélange que l'on homogénéise sous agitation douce. On incorpore sous agitation 88,16g d'eau portée à 90°C. La microémulsion obtenue est ensuite ramenée à température ambiante et forme alors une microdispersion de particules à base de cire.

Diamètre moyen des particules de cire : 99nm.

### EXEMPLE B

On opère comme à l'exemple A, le tensio-actif étant ici constitué par 1g d'oléate de potassium et 1g d'oléate de sodium. La quantité d'eau utilisée (88g) est celle permettant d'obtenir 100g de microémulsion.

On obtient une microdispersion de cire.

Diamètre moyen des particules de cire : 71nm.

### EXEMPLE C

De façon analogue, on réalise une microdispersion contenant 15% de cire de Carnauba et 7,7% d'oléate de triéthanolamine.

Diamètre moyen des particules de cire : 73nm.

### EXEMPLE D

De façon analogue, on réalise une microdispersion de cire contenant 10% de cire de Carnauba et 1,8% de dodécylsulfate de sodium.

Diamètre moyen des particules de cire : 202nm.

En remplaçant le dodécylsulfate de sodium par 2,2% de dodécylbenzène sulfonate de sodium, le diamètre moyen des particules est 277mm.

EXEMPLE E

De façon analogue, on réalise des microdispersions de cire contenant 10% de cire de Carnauba et respectivement x% d'émulsionnant non ionique.

| Emulsionnant | x | Diamètre moyen (nm) |
|---|---|---|
| Brij 98 | 2,4 | 108 |
| | 3,6 | 67 |
| | 4,8 | 46 |
| Brij 78 | 4,2 | 61 |
| Brij 58 | 4,7 | 39 |
| Myrj 51 | 6,75 | 111 |
| Myrj 52 | 8,6 | 74 |
| Tween 40 | 5,3 | 81 |
| Tween 60 | 5,4 | 69 |
| Tween 80 | 5,4 | 66 |

Le Brij 78 est un octadécanol polyoxyéthyléné à 20 moles d'oxyde d'éthylène (OE)
Le Brij 98 est un octadécène-1-ol polyoxyéthyléné à 20 OE
Le Brij 58 est un hexadécanol polyoxyéthyléné à 20 OE
Le Myrj 51 est un acide stéarique polyoxyéthyléné à 30 OE
Le Myrj 52 ist un acide stéarique polyoxyéthyléné à 40 OE
Le Tween 40 est un palmitate de sorbitol polyoxyéthyléné à 20 OE
Le Tween 60 est un stéarate de sorbitol polyoxyéthyléné à 20 OE
Le Tween 80 est un olérate de sorbitol polyoxyéthyléné à 20 OE.

EXEMPLE F

De façon analogue, on réalise des microdispersions à 15% de cire de Carnauba et respectivement x% d'émulsionnant non ionique.

| Emulsionnant | x | Diamètre moyen (nm) |
|---|---|---|
| Brij 98 | 3,6 | 102 |
| | 7,2 | 52 |
| | 10,8 | 34 |

EXEMPLE G

De façon analogue, on réalise une microdispersion de cire contenant 20% de cire de Carnauba et 4,8% de Brij 98.

Diamètre moyen des particules : 108nm.

EXEMPLE H

De façon analogue, on réalise une microdispersion de cire contenant 25% de cire de Carnauba et 11,95% d'émulsionnant non ionique Brij 98.

Diamètre moyen des particules : 36nm.

EXEMPLE I

De façon analogue on prépare des microdispersions de cire contenant 10%, 15% ou 20% de cire de Carnauba et respectivement x% d'émulsionnant non ionique (NI)

| Cire | Emulsionnant | x | Diamètre moyen (nm) |
|---|---|---|---|
| 10% | NI 1 | 2,7 | 104 |
| " | NI 2 | 2,9 | 147 |
| " | NI 3 | 3,0 | 106 |
| " | NI 4 | 3,2 | 190 |
| " | NI 5 | 1,8 | 96 |
| " | NI 6 | 5,2 | 257 |
| " | NI 7 | 5,4 | 343 |
| " | NI 8 | 4,74 | 314 |
| 15% | NI 2 | 4,4 | 171 |
| " | NI 3 | 4,5 | 131 |
| " | NI 4 | 4,85 | 213 |
| " | NI 9 | 2,8 | 145 |
| " | NI 5 | 2,1 | 145 |
| " | | 2,8 | 105 |
| " | | 3,45 | 80 |
| " | | 4,1 | 78 |
| 20% | NI 2 | 5,9 | 211 |
| " | NI 5 | 3,7 | 96 |

Le composé NI 1 est l'alcool oléylique polyglycérolé à 5 moles de glycérol (G).

Le composé NI 2 est l'alcool oléocétylique polyglycérolé à 6G.

Le composé NI 3 est l'alcool isostéarylique polyglycérolé à 6G.
Le composé NI 4 est l'octadécanediol-1,2 polyglycérolé à 7G.
Le composé NI 5 est le nonylphénol polyglycérolé à 3,25G.
Le composé NI 6 est le 1-octyléther d'hexadécanediol-1,2 polyglycérolé à 12G.
Le composé NI 7 est le 1-octyléther d'octadécanediol-1,2 polyglycérolé à 12G.
Le composé NI 8 est le 1-octyléther d'octadécanediol-1,3 polyglycérolé à 10G.
Le composé NI 9 est le nonylphénol polyglycérolé à 3G.

EXEMPLE J

De façon analogue, on prépare des microdispersions contenant de la cire de Candelilla (10%) et respectivement x% d'émulsionnant.

| Emulsionnant | x | Diamètre moyen (nm) |
|---|---|---|
| Oléate de potassium | 2,0 | 139 |
| Brij 98 | 4,8 | 289 |
| NI 5 | 1,8 | 157 |

EXEMPLE K

On utilise comme produit de départ une cire auto émulsionnable commercialisée par la Société TISCCO sous la dénomination CIRE AUTOLUSTRANTE OFR, qui est constituée d'un mélange de cire de Carnauba et de cire de paraffine en présence d'agents émulsionnants non ioniques.

On chauffe 12g du produit de départ vers 90°C en homogénéisant. On ajoute ensuite, en agitant, 88g d'eau à la même température.

On laisse ensuite refroidir à température ambiante la micro- dispersion de cire obtenue.

Diamètre moyen des particules : 150 nm.

EXEMPLE L

On utilise comme produit de départ une cire auto-émulsionnable commercialisée par la Société LA CERESINE sous la dénomination CERAX A.O. 28/B, qui est constituée d'une cire végétale d'Alfa en mélange avec un agent émulsionnant non ionique.

On chauffe 120g de ce mélange vers 90°C, en homogénéisant par agitation douce. On incorpore ensuite sous agitation 880g d'eau à température de 90°C environ.

On laisse ensuite refroidir à température ambiante la microdispersion de cire obtenue.

Diamètre moyen des particules : 250nm

EXEMPLE M

On a préparé de façon analogue à celle décrite à l'exemple A, des microdispersions de cire ayant les compositions suivantes :

| | |
|---|---|
| Cire de Carnauba .................. | x % |
| Huile ............................. | y % |
| Oléate de potassium ............... | 3,33 % |
| Eau ............................... | 86,67 % |

avec x + y = 10.

Les résultats sont résumés dans le tableau suivant.

| Ex | Cire x | Huile y | Diamètre moyen (nm) |
|---|---|---|---|
| | Carnauba | de paraffine | |
| M1 | 9 | 1 | 47 |
| M2 | 7 | 3 | 68 |
| | Carnauba | DV* | |
| M3 | 9 | 1 | 55 |
| M4 | 7 | 3 | 77 |
| | Carnauba | Tournesol | |
| M5 | 9 | 1 | 55 |
| M6 | 7 | 3 | |

* DV : (Ethyl 2 hexyloxy)-3 hexadecanoyloxy-1 propanol-2 décrit dans le brevet FR 2222351.

EXEMPLE N

De façon analogue, on a préparé des microdispersions de cire ayant la composition pondérale suivante :

| | |
|---|---|
| Cire de Carnauba .................... | 10 % |
| Additif lipophile .................. | x % |
| Brij 58 ............................ | 2,5 % |
| Eau ........................ qsp .. | 100 % |

La microdispersion est préparée suivant l'exemple A. L'additif lipophile est mélangé avec les autres ingrédients avant l'addition d'eau.

| EX | Additif lipophile x = | Diamètre moyen (nm) |
|---|---|---|
| N1 | Parsol MCX 3 % | 141 |
| N2 | { Parsol MCX 2,4%<br>Uvinul M40 0,6% | 132 |

- Le Parsol MCX est la dénomination commerciale de l'octyl Méthoxycinnamate vendu par GIVAUDAN
- L'Uvinul M40 est la dénomination commerciale de la benzophénone-3 vendue par BASF.

EXEMPLE O

On a préparé des microdispersions de cire ayant la composition pondérale suivante :

| | |
|---|---|
| Cire de Carnauba .................. | x % |
| Huile ............................. | y % |
| Brij 98 ........................... | 4,78% |
| Eau ............................... | 85,22% |

avec x + y = 10

Les microdispersions sont préparées selon l'exemple A. L'huile est ajoutée à la cire avant l'introduction de l'eau.

| EX | Cire x | Huile y | Diamètre moyen (nm) |
|---|---|---|---|
| | Carnauba | de paraffine | |
| 01 | 9,5 | 0,5 | 46 |
| 02 | 9 | 1 | 46 |
| 03 | 8,5 | 1,5 | 46 |
| 04 | 8 | 2 | 49 |
| 05 | 7,5 | 2,5 | 49 |
| 06 | 7 | 3 | 48 |
| | Carnauba | DV* | |
| 07 | 9 | 1 | 48 |
| 08 | 7 | 3 | 72 |
| | Carnauba | de Tournesol | |
| 09 | 9 | 1 | 52 |
| 010 | 7 | 3 | 290 |

* Voir exemple M

EXEMPLE P

On a préparé des microdispersions de cire ayant la composition pondérale suivante :

| | |
|---|---|
| Cire de Carnauba .................. | x % |
| Céramide et/ou cholestérol ........ | y % |
| Oléate de potassium ............... | 3,33% |
| Eau ............................... | 86,67% |

avec x + y = 10

Ces microdispersions sont préparées selon l'exemple A.

| EX | Cire x | Composé lipophile | Diamètre moyen (nm) |
|---|---|---|---|
| P1 | Carnauba 8 | DVA 2 | 108 |
| P2 | Carnauba 9 | Cholestérol 1 | 118 |
| P3 | 8 | 2 | 218 |
| P4 | Carnauba 8 | + DVA : 1<br>+ Cholestérol : 1 | 167 |

DVA : Céramide de formule :

$$C_{15}H_{31}CHOH\ \underset{\displaystyle NHCOC_{15}H_{31}}{\underset{|}{C}}HCH_2OH$$

mélange érythro : thréo

<u>EXEMPLE Q</u>

De façon analogue on a préparé des microdispersions de cire ayant la composition pondérale suivante :

| | |
|---|---|
| Carnauba ........................ | x % |
| Céramide ou cholestérol ......... | y % |
| Brij 58 ......................... | 2,34% |
| Eau ............................. | 87,66% |

avec x + y = 10

| EX | Cire x | Composé lipophile | Diamètre moyen (nm) |
|---|---|---|---|
| | Carnauba | DVB | |
| Q1 | 9 | 1 | 105 |
| Q2 | 8 | 2 | 127 |
| Q3 | 7 | 3 | 170 |
| | Carnauba | DVA | |
| Q4 | 8 | 2 | 92 |
| | Carnauba | Cholestérol | |
| Q5 | 9 | 1 | 136 |

DVA : Voir exemple P
DVB : Céramide de même formule que DVA, mais forme érythro seule.

EXEMPLE R

De façon analogue, on a préparé des microdispersions de cire ayant la composition pondérale suivante :

| | |
|---|---|
| Carnauba ........................ | x % |
| Céramide et cholestérol ......... | y % |
| Brij 58 ......................... | 2,34% |
| Eau ............................. | 87,66% |

avec x + y = 10

| EX | Cire x | Composé lipophile | Diamètre moyen (nm) |
|---|---|---|---|
| | Carnauba | DVA : 1 | |
| R | 8 | +Cholestérol:1 | 96 |

EXEMPLE S

De façon analogue, on a préparé des microdispersions ayant la composition pondérale suivante :

| | | | |
|---|---|---|---|
| Carnauba | | 10 | % |
| Additif lipophile | | x | % |
| Oléate de potassium | | 3,33 | % |
| Eau | qsp | 100 | % |

| EX | Additif lipophile x | Diamètre moyen (nm) |
|---|---|---|
| S1 | Colorant * 1 % | 43 |
| S2 | Parsol MCX 3 % | 254 |
| S3 | Parsol MCX 2,4% <br> Uvinul M40 0,6 % | 155 |

* Isopropyl-2 nitro-6 aniline.

EXEMPLES DE PREPARATION DE COMPOSITIONS COSMETIQUES

EXEMPLE 1

On prépare la composition de soin avant-shampooing suivante, en incorporant dans la microdispersion de cire les autres constituants, dans l'ordre indiqué :

- Microdispersion de cire obtenue à l'exemple L ... 98 g
- Hydroxypropylméthylcellulose vendue par la Société DOW CHEMICAL sous la dénomination METHOCEL F4M 1,5 g
- p.hydroxybenzoate de méthyle 0,2 g
- Dérivé d'imidazolidinylurée vendu sous la dénomination GERMALL 115 par la Société SUTTON LABS 0,3 g

On applique cette composition sur cheveux secs et non lavés, raie par raie sur les racines des cheveux. On laisse en contact 2 à 5 minutes, puis on procède au rinçage à l'eau et à un shampooing.

La quantité appliquée est de 2g/tête environ.

L'application de cette composition avant-shampooing confère à la chevelure du ressort, de la brillance, de la douceur et du volume.

EXEMPLE 2

On prépare le gel fluide de coiffage suivant, en incorporant dans la microdispersion de cire les autres constituants, dans l'ordre indiqué :

| | |
|---|---|
| - Microdispersion de cire obtenue à l'exemple L............................ | 20 g |
| - Acide polyacrylique réticulé (PM : 1 250 000) vendu par la société GOODRICH sous la dénomination CARBOPOL 941...................... | 1 g |
| - Dérivé d'imidazolidinylurée vendu sous la dénomination GERMALL 115 par la société SUTTON LABS...................... | 0,3 g |
| - Hydroxyde de sodium qs pH : 7 | |
| - Eau ..................................qsp | 100 g |

On applique cette composition sur cheveux propres et secs, sur les racines et sur la longueur des cheveux, à raison de 2 à 5 g par tête selon l'abondance de la chevelure.

Le produit s'étale facilement et de manière homogène dans les cheveux, leur confère de la douceur et du volume, du gonflant et de la discipline.

EXEMPLE 3

On prépare la lotion coiffante suivante, en incorporant dans la microdispersion de cire les autres constituants, dans l'ordre indiqué :

| | |
|---|---|
| - Microdispersion à 10% de cire de Carnauba préparée selon l'exemple A..... | 99,65 g |
| - p.hydroxybenzoate de méthyle............... | 0,15 g |
| - Dérivé d'imidazolidinylurée vendu sous la dénomination GERMALL 115 par la société SUTTON LABS...................... | 0,20 g |

On applique cette composition sur cheveux propres et secs, à partir des racines et sur la longueur des cheveux, à raison de 2g/tête (pour une chevelure féminine moyenne).

Les cheveux ainsi traités ont du volume, de l'épaisseur, de la tenue et sont disciplinés. Leur brossage révèle tout leur gonflant.

EXEMPLE 4

On prépare la lotion coiffante suivante, en incorporant dans la microdispersion de cire les autres constituants, dans l'ordre indiqué :

- Microdispersion à 10% de cire de Carnauba et 2,7% de NI 1 (voir exemple I)................ 99,65 g
- Parahydroxybenzoate de méthyle.................. 0,15 g
- Dérivé d'imidazolidinylurée vendu sous la dénomination GERMALL 115 par la société SUTTON LABS.................................... 0,20 g

Cette composition s'utilise en finition de coiffage, sur cheveux secs et propres. L'application est faite sur la racine des cheveux avec répartition sur la longueur des cheveux, à raison de 2 g/tête.

Les cheveux sont brillants, gonflants et légers.

EXEMPLE 5

On prépare la composition de traitement avant-shampooing suivante, en incorporant dans la microdispersion de cire les autres constituants, dans l'ordre indiqué :

- Microdispersion à 10% de cire de Carnauba et 2,9% de NI 2 (voir exemple I)................... 97,5 g
- Hydroxypropylcellulose commercialisée sous la dénomination KLUCEL H par la société HERCULES... 2 g
- Parahydroxybenzoate de méthyle.................. 0,2 g
- Dérivé d'imidazolidinylurée vendu sous la dénomination GERMALL 115 par la société SUTTON LABS.................................... 0,3 g

On applique cette composition (environ 2g/tête) raie par raie sur des cheveux secs non lavés et on laisse en contact pendant 5 minutes avant d'effectuer un shampooing.

L'application de cette composition confère aux cheveux du volume (décollement des racines), du lissage et de la brillance.

EXEMPLE 6

On prépare la lotion structurante suivante, en incorporant dans la microdispersion de cire les autres constituants, dans l'ordre indiqué :

- Microdispersion à 10% de cire de Carnauba et 3% de NI 3 (voir exemple I) .................... 99,5 g
- Parahydroxybenzoate de méthyle.................. 0,2 g
- Dérivé d'imidazolidinylurée vendu sous la dénomination GERMALL 115 par la Société SUTTON LABS.................................... 3 g

Cette composition est destinée à la finition du coiffage; elle est appliquée par conséquent sur cheveux secs, propres, à raison de 2 à 3 g par tête suivant l'abondance de la chevelure.

Sa vitesse de séchage rapide et sa répartition principalement sur les racines des cheveux donnent à la coiffure structure, volume et discipline.

EXEMPLE 7

On prépare un gel fluide de coiffage ayant la composition suivante, en incorporant dans la microdispersion de cire les autres constituants, dans l'ordre indiqué :

- Microdispersion de cire obtenue
  à l'exemple K................................ 10 g
- CARBOPOL 941................................ 1,5 g
- NaOH........................................ 0,6 g
- PEG-15 COCAMINE............................. 3 g
- GERMALL 115................................. 0,2 g
- p-hydroxybenzoate de méthyle................ 0,2 g

- Sorbate de potassium........................ 0,3 g
- Parfum , ........q.s.
- Triéthanolamine, q.s. pH = 7
- Eau...............................q.s.p.. 100 g

PEG-15 COCAMINE : Polyéthylèneglycol amine d'acide de coprah, selon la définition de la CTFA (Cosmetic, Toiletry and Fragrance Association); produit vendu par AKZO sous la dénomination commerciale ETHOMEEN C25.

Ce gel est utilisé comme celui de l'exemple 2.

EXEMPLE 8

Cette lotion est préparée en mélangeant dans l'ordre les constituants suivants :

- Microdispersion de cire obtenue à l'exemple M1 .. 95 g
- para hydroxybenzoate de méthyle ................. 0,15g
- dérivé d'imidazolidinylurée vendu sous la
  dénomination GERMALL 115 par la Société
  SUTTON LABS ..................................... 0,2 g
- NaOH = qsp pH 6,8
- Eau qsp ......................................... 100 g

La composition est appliquée sur des cheveux secs et propres. Les cheveux traités sont brillants et présentent du gonflant.

EXEMPLE 9

On opère, comme dans l'exemple 1, avec les constituants suivants :

- Microdispersion de cire obtenue à l'exemple M4 .. 98 g
- para hydroxybenzoate de méthyle ................ 0,1 g
- GERMALL 115 ..................................... 0,25g
- NaOH qsp pH7
- eau qsp ......................................... 100 g

On applique cette composition après un shampoing. Les cheveux traités sont brillants et disciplinés.

EXEMPLE 10

Cette lotion est préparée en mélangeant dans l'ordre les constituants suivants:

- Microdispersion de cire obtenue à l'exemple N2 ..........................99,65g
- para hydroxybenzoate de méthyle ................ 0,15g
- GERMALL 115 ..................................... 0,20
- NaOH qsp pH7

L'application est faite sur cheveux secs et propres. Outre les effets de volume, de tenue et de discipline, la composition apporte aux cheveux un effet de protection vis-à-vis des rayons UV.

EXEMPLE 11

On opère, comme dans l'exemple 1, avec les constituants suivants :

- Microdispersion de cire obtenue à l'exemple O7 .. 95 g
- para hydroxybenzoate de méthyle ................ 0,15 g
- GERMALL 115 ..................................... 0,2 G
- NaOH qsp pH 6,9
- eau qsp ......................................... 100 g

Cette composition s'applique comme à l'exemple 9. On obtient sur les cheveux des résultats analogues.

EXEMPLE 12

Cette lotion est préparée en mélangeant dans l'ordre les constituants suivants :

- Microdispersion de cire obtenue à l'exemple P4 .. 50 g
- para hydroxybenzoate de méthyle ................ 0,15g
- GERMALL 115 ..................................... 0,2
- NaOH qsp pH7
- eau qsp ......................................... 100 g

Cette composition s'utilise après un shampoing. Elle apporte un effet de protection. Les cheveux présentent du corps et du volume.

EXEMPLE 13

On opère comme à l'exemple précédent, en remplaçant la microdispersion de l'exemple R4 par celle de l'exemple Q5. On obtient des résultats analogues.

EXEMPLE 14

Cette lotion est préparée en mélangeant dans l'ordre les constituants suivants :

- Microdispersion de cire obtenue à l'exemple S1 .. 98 g
- para hydroxybenzoate de méthyle ................. 0,1 g
- GERMALL 115 ..................................... 0,1
- NaOH qsp pH6,8
- eau qsp ......................................... 100 g

Cette composition est appliquée comme dans l'exemple 3. On obtient des effets analogues sur la chevelure qui, de plus, est colorée en jaune.

**Revendications**

1. Utilisation, comme composition cosmétique ou support de composition cosmétique pour cheveux, d'une composition fluide non moussante contenant une dispersion de cire dans un véhicule liquide, dont la phase dispersée est une microdispersion stable de particules solides de dimensions inférieures à 500nm, lesdites particules contenant une cire ou un mélange de cires et éventuellement une huile à une concentration pouvant aller jusqu'à 30% en poids, par rapport à la cire, ladite cire ou ledit mélange ayant un point de fusion finissante supérieur à 60°C et inférieur à 100°C et étant capable de former une microdispersion telle que définie ci-dessus, ladite composition contenant, en poids, de 0,1 à 40% de cire, de 0,01 à 25% d'au moins un agent émulsionnant non ionique ou anionique et au moins 35% d'eau, le rapport pondéral cire/émulsionnant pouvant varier de 1 à 30, et ladite composition ne contenant pas d'agent tensioactif cationique.

2. Utilisation selon la revendication 1, caractérisée par le fait que ladite cire est choisie parmi la cire de Carnauba, la cire de Candelilla, la cire d'Alfa et leurs mélanges.

3. Utilisation selon la revendication 1, caractérisée par le fait que la cire contient, outre de la cire de Carnauba ou de la cire de Candelilla ou leurs mélanges, une autre cire ou un mélange d'autres cires.

4. Utilisation selon la revendication 3, caractérisée par le fait que ladite autre cire est une cire de paraffine, l'ozokérite, la cire de jojoba hydrogénée, la cire de riz, la cire d'abeille éventuellement estérifiée, ou un céramide.

5. Utilisation selon l'une quelconque des revendications 3 et 4, caractérisée par le fait que la proportion pondérale de cire de Carnauba et/ou de Candelilla, dans de tels mélanges, est supérieure ou égale à 50%.

6. Utilisation selon l'une quelconque des revendications précédentes, caractérisée par le fait que l'agent émulsionnant est présent à une concentration de 0,1 à 10%.

7. Utilisation selon l'une quelconque des revendications précédentes, caractérisée par le fait que ledit agent émulsionnant non ionique est un tensio-actif polyalcoxylé ou polyglycérolé.

8. Utilisation selon l'une quelconque des revendications précédentes, caractérisée par le fait que ledit agent émulsionnant est un tensio-actif anionique.

9. Utilisation selon l'une quelconque des revendications précédentes, caractérisée par le fait que ledit véhicule aqueux est constitué par de l'eau.

10. Utilisation selon l'une quelconque des revendications précédentes, caractérisée par le fait que le rapport pondéral cire/émulsionnant est dans la gamme de 1 à 20.

11. Utilisation selon la revendication 10, caractérisée par le fait que ledit rapport varie de 2 à 10.

12. utilisation selon l'une quelconque des revendications précédentes, caractérisée par le fait qu'au moins un composé amphiphile non émulsionnant des cires est associé à la cire, à une concentration pouvant aller jusqu'à 30 %, en particulier jusqu'à 10 % en poids par rapport à la cire.

13. Utilisation selon la revendication 12, caractérisée par le fait que ledit composé amphiphile est choisi parmi le cholestérol et les alcools gras ayant au moins 12 atomes de carbone.

14. Utilisation selon l'une quelconque des revendications précédentes, caractérisée par le fait qu'une huile ou un mélange d'huiles est associé à la cire, à une concentration pouvant aller jusqu'à 10 % en poids, par rapport à la cire.

15. Utilisation selon l'une quelconque des revendications précédentes, caractérisée par le fait qu'au moins un ingrédient actif liposoluble est associé à la cire, à une concentration pouvant aller jusqu'à 30 %, en particulier jusqu'à 10 %, en poids par rapport à la cire.

16. Utilisation selon la revendication 15, caractérisée par le fait que ledit ingrédient actif liposoluble est choisi parmi les colorants et les filtres solaires.

17. Utilisation, comme composition cosmétique, d'une composition telle que définie dans l'une quelconque des revendications précédentes, caractérisée par le fait que ladite composition contient de 1 à 20% de cire, et en particulier de 1 à 10% de cire.

18. Utilisation selon l'une quelconque des revendications 1 à 13 et 15 à 17, caractérisée par le fait que ladite composition ne contient pas d'huile.

19. Procédé de traitement cosmétique des cheveux destiné à améliorer la tenue et le volume de la coiffure et/ou à supprimer ou à retarder l'apparition de l'aspect gras des cheveux, caractérisé par le fait que l'on applique, au moins sur la partie des cheveux voisine de la racine, en quantité suffisante pour imprégner les cheveux ou les parties de cheveux à traiter, une composition telle que définie dans l'une quelconque des revendications précédentes, après dilution éventuelle pour que la proportion pondérale de cire soit de 0,1 à 20%.

## Claims

1. Use, as a cosmetic composition or carrier for a cosmetic composition for hair, of a non-foaming fluid composition containing a dispersion of wax in a liquid vehicle, the disperse phase of which is a stable microdispersion of solid particles less than 500 nm in size, the said particles containing wax or a mixture of waxes, and optionally an oil at a concentration which can range up to 30 % by weight relative to the wax, the said wax or said mixture having a final melting point above 60°C and below 100°C and being capable of forming a microdispersion as defined above, the said composition containing, by weight, from 0.1 to 40 % of wax, from 0.01 to 25 % of at least one nonionic or anionic emulsifying agent and at least 35 % of water, it being possible for the wax/emulsifier weight ratio to vary from 1 to 30, and the said composition not containing a cationic surfactant.

2. Use according to Claim 1, characterised in that the said wax is chosen from carnauba wax, candelilla wax, alfa wax and mixtures thereof.

3. Use according to Claim 1, characterised in that the wax contains, apart from carnauba wax or candelilla wax or mixtures thereof, another wax or a mixture of other waxes.

4. Use according to Claim 3, characterised in that the said other wax is a paraffin wax, ozocerite, hydrogenated jojoba wax, rice wax, beeswax, optionally esterified, or a ceramide.

5. Use according to either of Claims 3 and 4, characterised in that the weight proportion of carnauba and/or

candelilla wax in such mixtures is not less than 50 %.

6. Use according to any one of the preceding claims, characterised in that the emulsifying agent is present at a concentration of 0.1 to 10 %.

7. Use according to any one of the preceding claims, characterised in that the said nonionic emulsifying agent is a polyalkoxylated or polyglycerolated surfactant.

8. Use according to any one of the preceding claims, characterised in that the said emulsifying agent is an anionic surfactant.

9. Use according to any one of the preceding claims, characterised in that the said aqueous vehicle consists of water.

10. Use according to any one of the preceding claims, characterised in that the wax/emulsifier weight ratio is in the range from 1 to 20.

11. Use according to Claim 10, characterised in that the said ratio varies from 2 to 10.

12. Use according to any one of the preceding claims, characterised in that at least one amphiphilic compound which does not emulsify the waxes is combined with the wax, at a concentration which can range up to 30 %, and especially up to 10 %, by weight relative to the wax.

13. Use according to Claim 12, characterised in that the said amphiphilic compound is chosen from cholesterol and fatty alcohols having at least 12 carbon atoms.

14. Use according to any one of the preceding claims, characterised in that an oil or a mixture of oils is combined with the wax, at a concentration which can range up to 10 % by weight relative to the wax.

15. Use according to any one of the preceding claims, characterised in that at least one fat-soluble active ingredient is combined with the wax, at a concentration which can range up to 30 %, and especially up to 10 %, by weight relative to the wax.

16. Use according to Claim 15, characterised in that the said fat-soluble active ingredient is chosen from colorants and sunscreen agents.

17. Use of a composition as defined in any one of the preceding claims as a cosmetic composition, characterised in that the said composition contains from 1 to 20 % of wax, and especially from 1 to 10 % of wax.

18. Use according to any one of Claims 1 to 13 and 15 to 17, characterised in that the said composition does not contain an oil.

19. Process for the cosmetic treatment of hair, intended for improving the hold and volume of the hairstyle and/or for eliminating or retarding the onset of a greasy appearance of the hair, characterised in that a composition as defined in any one of the preceding claims is applied, after dilution, where appropriate, so that the weight proportion of wax is from 0.1 to 20 %, at least to the part of the hair close to the root, in an amount sufficient to impregnate the hair or the hair parts to be treated.

**Patentansprüche**

1. Verwendung einer nicht schäumenden Zusammensetzung als kosmetische Zusammensetzung oder Träger für eine kosmetische Zusammensetzung für Haare, die eine Wachsdispersion in einem flüssigen Träger enthält, in dessen Phase eine stabile Mikrodispersion von festen Teilchen mit Dimensionen unterhalb von 500 nm dispergiert ist, wobei die Teilchen ein Wachs oder eine Mischung von wachsen und gegebenenfalls ein Öl in einer Konzentration bim zu 30 Gew.-%, bezogen auf das wachs, enthalten, und wobei das Wachs oder die Mischung einen Schmelzpunkt besitzt, der oberhalb von 60°C und unterhalb von 100°C liegt, und das dazu fähig ist, eine wie vorstehend definierte Mikrodispersion zu bilden, wobei die Zusammensetzung, in Gewichtsprozent, 0.1 bis 40 % Wachs, von 0.01 bis 25 % mindestens eines nichtionischen und anionischen Emulgiermittels oder mindestens 35 % Wasser enthält, wobei das Gewichtsverhältnis wachs/Emulgiermittel zwischen 1 und 30 variieren kann, und die Zusammensetzung kein kationisches oberflächenaktives Mittel enthält.

2. Verwendung nach Anspruch 1, dadurch gekennzeichnet, daß das Wachs ausgewählt ist aus Carnaubawachs, Candelillawachs, Alfawachs und ihren Mischungen.

3. Verwendung nach Anspruch 1, dadurch gekennzeichnet, daß das wachs außer Carnaubawachs oder dem Kandelillawachs oder ihren Mischungen ein anderes Wachs oder eine Mischung aus anderen Wachsen enthält.

4. Verwendung nach Anspruch 3, dadurch gekennzeichnet, daß das andere Wachs ein Paraffinwachs ist, Ozokerit, hydriertes Jojobawachs, Reiswachs, gegebenenfalls verestertes Bienenwachs, oder ein Steinwachs ist.

5. Verwendung nach einem der Ansprüche 3 und 4, dadurch gekennzeichnet, daß das Gewichtsverhältnis von Carnaubawachs und/oder Candelillawachs in diesen Mischungen größer oder gleich 50 % beträgt.

6. Verwendung nach einem der vorhergehenden Ansprüche, dadurch gekennzeichnet, daß das Emulgiermittel in einer Konzentration von 0.1 bis 10 % vorhanden ist.

7. Verwendung nach einem der vorhergehenden Ansprüche, dadurch gekennzeichnet, daß das nichtionische Emulgiermittel ein oberflächenaktives Polyalkoxyl oder Polyglycerin ist.

8. Verwendung nach einem der vorhergehenden Ansprüche, dadurch gekennzeichnet, daß das Emulgiermittel ein anionisches oberflächenaktives Mittel ist.

9. Verwendung nach einem der vorhergehenden Ansprüche, dadurch gekennzeichnet, daß der wässerige Träger aus Wasser besteht.

10. Verwendung nach einem der vorhergehenden Ansprüche, dadurch gekennzeichnet, daß das Gewichtsverhältnis Wachs/Emulgiermittel im Bereich von 1 bis 20 liegt.

11. Verwendung nach Anspruch 10, dadurch gekennzeichnet, daß das Gewichtsverhältnis von 2 bis 10 variiert.

12. Verwendung nach einem der vorhergehenden Ansprüche, dadurhc gekennzeichnet, daß mindestens eine amphiphile nicht emulgierende wachsartige Verbindung mit dem Wachs in einer Konzentration bis zu 30 % vorhanden sein kann, und inbesondere bis zu 10 Gew.-%, bezogen auf das Wachs.

13. Verwendung nach Anspruch 12, dadurch gekennzeichnet, daß die amphiphile Verbindung ausgewählt ist aus Cholesterin und den Fettalkoholen mit mindestens 12 Kohlenstoffatomen.

14. Verwendung nach einem der vorhergehenden Ansprüche, dadurch gekennzeichnet, daß ein Öl oder eine Mischung von Ölen zusammen mit dem Wachs in einer Konzentration bis zu 10 Gew.-%, bezogen auf das Wachs, vorhanden ist.

15. Verwendung nach einem der vorhergehenden Ansprüche, dadurch gekennzeichnet, daß zusammen mit dem Wachs mindestens ein fettlöslicher aktiver Bestandteil in einer Konzentration bis zu 30, insbesondere bis zu 10 Gew.-%, besogen auf das Wachs, vorhanden ist.

16. Verwendung nach Anspruch 15, dadurch gekennzeichnet, daß der fettlösliche aktive Bestandteil ausgewählt ist unter den Farbstoffen und den Sonnenfiltern.

17. Verwendung einer in den vorstehenden Ansprüchen definierten Zusammensetzung als kosmetische Zusammensetzung, dadurch gekennzeichnet, daß die Zusammensetzung 1 bis 20 % Wachs, und insbesondere 1 bis 10 % Wachs enthält.

18. Verwendung nach einem der Ansprüche 1 bis 13 und 15 bis 17, dadurch gekennzeichnet, daß die Zusammensetzung kein Öl enthält.

19. Verfahren zur kosmetischen Behandlung von Haar zur Verbesserung des Haltes und der Fülle der Frisur

und/oder zum Entfetten oder der Vermeidung von fettigem Aussehen von Haar, dadurch gekennzeichnet, daß man mindestens auf ein Teil der den Wurzeln benachbarten Haare eine ausreichende Menge einer Zusammensetzung, wie sie in einem der vorstehenden Ansprüche definiert ist, zur Imprägnierung der Haare oder der zu behandelnden Haarpartien aufbringt, und gegebenenfalls danach auf einen Gewichtsanteil des Wachses von 0.1 bis 20 % verdünnt.